# DEMANDE DE BREVET EUROPEEN

(11) **EP 1 714 957 A1**
(43) Date de publication de la demande: **25.10.2006**
(21) Numéro de dépôt: 06300218.2
(22) Date de dépôt: 10.03.2006
(51) Int. Cl.: C07C 67/03, C11C 3/10

(54) **Procédé de purification par lavage à l'eau des phases liquides issues d'une réaction de transestérification**

(30) Priorité: 22.04.2005 FR 0551030
(71) Demandeur: L'Air Liquide Société Anonyme à Directoire et Conseil de Surveillance pour l'Etude et l'Exploitation des Procédés Georges Claude, 75321 Paris Cedex 07 (FR)
(72) Inventeur: De Rigaud, Jean-Mathieu, 78000 VERSAILLES (FR); Perru, Laurent M., 78780 MAURECOURT (FR)
(74) Mandataire: Ducreux, Marie

(57) **Abrégé**

L'invention concerne un procédé de purification par lavage à l'eau des phases liquides issues d'une réaction de transestérification, dans lequel le lavage des phases liquides est réalisé en présence d'anhydride carbonique.

## Description

La présente invention concerne un procédé de purification des phases liquides issues d'une réaction de transestérification, notamment mis en oeuvre lors de la synthèse de biodiesel.

Le biodiesel est un ester produit par réaction de transestérification entre des alcools et des huiles, qui peuvent être des huiles végétales (principalement de colza), mais aussi des huiles de cuisson ou même des graisses animales. L'utilisation directe locale de l'huile brute dans les tracteurs est intéressante pour les agriculteurs, mais l'ester issu de l'huile brute (ou biodiesel) est de meilleure qualité ; il est moins visqueux et il s'auto-enflamme mieux que l'huile brute. Le biodiesel a un meilleur rendement que l'éthanol.

La réaction de synthèse du biodiesel consiste à faire réagir : les huiles essentiellement végétales avec au moins un alcool, généralement le méthanol, et un ester en présence d'un catalyseur, par exemple de l'hydroxyde de potassium. Cet ester est généralement obtenu par réaction d'estérification entre des huiles de recyclage et du méthanol en présence d'acide sulfurique comme catalyseur. La réaction de transestérification est habituellement réalisée en phase liquide : elle conduit à deux phases liquides, l'une comprenant majoritairement l'ester recherché, le biodiesel, et l'autre phase comprenant de la glycérine. La phase liquide comprenant majoritairement le biodiesel est purifiée pour en éliminer les traces de méthanol n'ayant pas réagi, le catalyseur et les savons issus de l'étape de transesterification. Cette purification consiste essentiellement en des cycles de lavage à l'eau propre, complétés éventuellement par une distillation. L'eau et l'alcool sont séparés de la glycérine. L'alcool et le méthanol qui sont retenus dans les eaux de lavage de la glycérine et du biodiesel, sont eux-mêmes lavés à l'eau. La phase liquide comprenant majoritairement la glycérine brute subit généralement une coupure des chaînes carbonatées par action d'un acide fort du type acide chlohydrique, afin d'obtenir un savon adapté à la demande des marchés.

Au cours de ces lavages à l'eau des phases liquides issues de la transestérification, la quantité d'eau par cycle de lavage et le nombre de cycles pour obtenir une pureté optimale des produits finis biodiesel et/ou glycérine, influe sur les paramètres de productivité du procédé global.

Le but de la présente invention est de proposer un procédé de purification par lavage à l'eau des phases liquides issues d'une réaction de transestérification permettant de réduire la quantité d'eau utilisée ainsi que le nombre de cycles de lavage.

Dans ce but, l'invention concerne un procédé de purification par lavage à l'eau des phases liquides issues d'une réaction de transestérification, dans lequel on sépare les phases liquides à laver du milieu réactionnel de la transestérification et on lave lesdites phases liquides au moyen d'une eau de lavage en présence d'anhydride carbonique. L'invention consiste donc en l'ajout de l'anhydride carbonique dans l'étape de lavage des différentes phases liquides issues de la réaction de transestérification. L'invention est particulièrement adaptée au cas où la réaction de transestérification est une réaction de synthèse de biodiesel à partir d'alcools, d'esters et d'huiles. Dans ce cas particulier, le procédé selon l'invention peut s'appliquer tout d'abord à la phase liquide comprenant majoritairement le biodiesel et à la phase liquide comprenant majoritairement la glycérine. Par phase liquide comprenant majoritairement un composé, on entend le fait que la phase liquide comprend au moins 50 % en poids de ce composé.

Selon une variante de l'invention, on lave la phase liquide comprenant majoritairement de la glycérine au moyen d'une eau de lavage comprenant de l'anhydride carbonique préalablement à une réaction de coupure des chaînes carbonatées de la glycérine au moyen d'un acide fort. Cette variante permet de réduire la quantité d'acide fort utilisé pour couper les chaînes et, par conséquent, la quantité de sels dans la glycérine finale. De cette façon, la glycérine est valorisée et son traitement ultérieur est simplifié. En effet, on connaît l'importance de la teneur en sels dans le produit fini sur les étapes de transformation ultérieures. Ce pourcentage, même faible, des sels issus des chlorures introduits lors de la coupure des chaînes carbonatées de la glycérine brute, gêne considérablement les opérations ultérieures en ralentissant la productivité ou nécessitant de sur-dimensionner les équipements. La présente variante permet de résoudre ce problème en diminuant l'apport d'ions chlorures de l'ordre de 50 à 70 %, c'est-à-dire en diminuant la formation de sels dans la glycérine finie du même pourcentage.

L'invention peut également s'appliquer à une phase liquide comprenant de l'alcool (principalement du méthanol) et issue :
- de la purification par lavage de la phase liquide comprenant majoritairement la glycérine, ou
- de la purification par lavage de la phase liquide comprenant majoritairement le biodiesel, ou
- de l'étape de séparation des phases liquides de biodiesel et de glycérine à la fin de la réaction de transestérification. En effet, suite au lavage de la phase liquide comprenant majoritairement la glycérine ou le biodiesel, on récupère une phase liquide comprenant de l'alcool n'ayant pas réagi au cours de la réaction de transestérification. Cet alcool doit être enlevé des flux pour éviter la réversibilité de la réaction de transestérification. Il est généralement réintroduit dans la réaction de transestérification. Ce lavage est mis en oeuvre selon le procédé de l'invention : de l'anhydride carbonique est alors introduit au cours du lavage.

Généralement, l'anhydride carbonique est introduit dans l'eau du lavage préalablement à la mise en contact de ladite eau avec la phase liquide à laver. En pratique, l'anhydride carbonique peut être injecté sur la boucle de recirculation de l'eau de lavage, ou sur l'arrivée de l'eau dite claire, ou sur un circuit d'eau dérivé aménagé spécifiquement pour faciliter cette introduction. Selon une variante particulièrement avantageuse de l'invention, l'anhydride carbonique est introduit à la sortie d'une pompe de circulation d'eau de lavage

On peut également introduire l'anhydride carbonique dans les phases liquides séparées à laver. Cette mise en oeuvre ne peut être appliquée que si la viscosité des phases liquide à laver le permet.

L'introduction de l'anhydride carbonique dans le procédé de lavage peut se faire de façon continu ou par batchs, selon la nature du procédé général de transestérification.

Lorsque l'on traite la phase liquide comprenant majoritairement le biodiesel ou la phase liquide comprenant majoritairement la glycérine, on introduit génralement entre 15 et 20 g de CO₂ par kg de biodiesel ou de glycérine contenu dans la phase liquide, de préférence environ 17 g de CO₂ par kg de biodiesel ou de glycérine contenu dans la phase liquide.

Lorsque l'on traite une des phases liquides comprenant de l'alcool et issue de la purification par lavage de la phase liquide comprenant majoritairement la glycérine, ou de la purification par lavage ou de la phase liquide comprenant majoritairement le biodiesel, ou de l'étape de séparation des phases liquides de biodiesel et de glycérine à la fin de la réaction de transestérification, on introduit de préférence entre 8 et 10 g de CO₂ par m³ de phase liquide traitée.

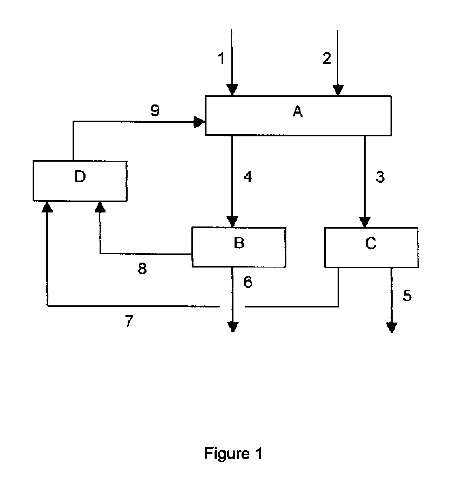

La figure 1 permet d'illustrer le fonctionnement de l'invention. L'étape de transestérification A est obtenue par mise en oeuvre par réaction entre les huiles 1, au moins un ester 2 et un alcool 9. Suite à cette réaction, les phases liquides obtenues sont séparées : l'une 3 comprend majoritairement le biodiesel, une autre 4 comprend majoritairement de la glycérine. La phase 3 est lavée au cours de l'étape C de purification par lavage, ce qui produit le biodiesel purifié 5 et une phase 7 comprenant les impuretés, dont l'alcool n'ayant pas réagi. La phase 4 est lavée au cours de l'étape B de purification par lavage, ce qui produit la glycérine purifiée 6 et une phase 8 comprenant les impuretés, dont l'alcool n'ayant pas réagi. Les deux phases 7, 8 comprenant les impuretés issues du lavage sont elles-mêmes lavées au cours de l'étape D de manière à produire de l'alcool purifiée 9 qui peut être recyclé dans la réaction de transestérification A. Selon l'invention, l'anhydride carbonique peut être injecté au cours d'au moins une des étapes suivantes :
- à la fin de l'étape A et préalablement à la séparation des phases liquides 3 et 4,
- dans les phases liquides 3 et 4,
- au cours des étapes B et C, par introduction dans l'eau de lavage,
- au cours de l'étape D, par introduction dans l'eau de lavage.

Par mise en oeuvre d'un procédé tel que précédemment décrit, différents avantages sont obtenus :
- du fait de la diminution de la consommation d'eau de lavage, les gains de productivité du procédé de lavage sont compris entre 5 et 20 %,
- en utilisant moins d'eau, le biodiesel purifié peut être séché plus rapidement,
- le dioxyde de carbone n'introduit aucun sel dans le procédé de type sulfate ou chlorure,
- un biodiesel plus pur que dans l'art antérieur peut être obtenu si la quantité d'eau et de cycles de lavage est maintenue.

## Revendications

1. Procédé de purification par lavage à l'eau des phases liquides (3, 4) issues d'une réaction de transestérification (A), **caractérisé en ce qu'**on sépare les phases liquides à laver du milieu réactionnel de la transestérification et on lave lesdites phases liquides au moyen d'une eau de lavage en présence d'anhydride carbonique.

2. Procédé de purification selon la revendication 1, **caractérisé en ce que** la réaction de transestérification (A) est une réaction de synthèse de biodiesel à partir d'alcools (9), d'esters (2) et d'huiles (1).

3. Procédé de purification selon la revendication 2, **caractérisé en ce que** les phases liquides à purifier sont la phase liquide (3) comprenant majoritairement le biodiesel et la phase liquide (4) comprenant majoritairement la glycérine.

4. Procédé selon la revendication 3, **caractérisé en ce qu'**on lave la phase liquide (4) comprenant majoritairement de la glycérine au moyen d'une eau de lavage comprenant de l'anhydride carbonique préalablement à une réaction de coupure des chaînes carbonatées de la glycérine au moyen d'un acide fort.

5. Procédé de purification selon la revendication 2, **caractérisé en ce qu'**une des phases liquides à purifier est une phase liquide (7, 8) comprenant de l'alcool et issue :
- de la purification (B) par lavage de la phase liquide (4) comprenant majoritairement la glycérine, ou
- de la purification (C) par lavage de la phase liquide (3) comprenant majoritairement le biodiesel, ou
- de l'étape de séparation des phases liquides de biodiesel et de glycérine à la fin de la réaction de transestérification (A).

6. Procédé de purification selon l'une des revendications précédentes, **caractérisé en ce que** l'anhydride carbonique est introduit dans l'eau du lavage préalablement à la mise en contact de ladite eau avec la phase liquide à laver.

7. Procédé de purification selon l'une des revendications 1 à 5, **caractérisé en ce qu'**on introduit l'anhydride carbonique dans les phases liquides séparées à laver.

8. Procédé de purification selon l'une des revendications précédentes, **caractérisé en ce qu'**on introduit l'anhydride carbonique sous forme gazeuse dans l'eau de lavage.

9. Procédé de purification selon la revendication 3, **caractérisé en ce qu'**on introduit entre 15 et 20 g de CO₂ par kg de biodiesel ou de glycérine contenu dans la phase liquide.

10. Procédé de purification selon la revendication 4, **caractérisé en ce qu'**on introduit entre 8 et 10 grammes de CO₂ par m³ de phase liquide traitée.
